(19) **Europäisches Patentamt / European Patent Office / Office européen des brevets**

(11) **EP 3 946 028 B1**

(12) # EUROPEAN PATENT SPECIFICATION

(45) Date of publication and mention
of the grant of the patent:
**21.08.2024 Bulletin 2024/34**

(21) Application number: **19724906.3**

(22) Date of filing: **28.03.2019**

(51) International Patent Classification (IPC):
**A61B 5/024** (2006.01)    **A61B 5/026** (2006.01)
**A61B 5/1455** (2006.01)    **A61B 5/00** (2006.01)

(52) Cooperative Patent Classification (CPC):
**A61B 5/0261; A61B 5/02416; A61B 5/02427;**
**A61B 5/02433; A61B 5/14552; A61B 5/6802;**
**A61B 5/6887; A61B 5/7203;** A61B 5/6803;
A61B 5/6804; A61B 5/6807; A61B 5/681;
A61B 5/6811; A61B 5/6815; A61B 5/6824;    (Cont.)

(86) International application number:
**PCT/IB2019/052552**

(87) International publication number:
**WO 2020/194036 (01.10.2020 Gazette 2020/40)**

(54) **PPG SENSOR HAVING A HIGH SIGNAL TO NOISE RATIO**

PPG-SENSOR MIT HOHEM SIGNAL-RAUSCH-VERHÄLTNIS

CAPTEUR DE PPG AYANT UN RAPPORT SIGNAL SUR BRUIT ÉLEVÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**09.02.2022 Bulletin 2022/06**

(73) Proprietor: **Aktiia SA**
**2000 Neuchâtel (CH)**

(72) Inventors:
• **DE MARCO, Bastien**
**1006 Lausanne (CH)**
• **CHAPUIS, Valentin**
**2300 La Chaux-de-Fonds (CH)**
• **VYBORNOVA, Anna**
**London SE15 5TF (GB)**
• **FALLET, Sibylle**
**1426 Corcelles-près-Concise (CH)**
• **GROSSENBACHER, Olivier**
**2000 Neuchâtel (CH)**
• **SIUTRYK, Nadège**
**1296 Coppet (CH)**

• **OLIVERO, Elisa**
**1429 Giez (CH)**
• **BERTSCHI, Mattia**
**1066 Epalinges (CH)**
• **SOLÀ I CARÓS, Josep Maria**
**2036 Cormondrèche (CH)**

(74) Representative: **P&TS SA (AG, Ltd.)**
**Avenue J.-J. Rousseau 4**
**P.O. Box 2848**
**2001 Neuchâtel (CH)**

(56) References cited:
EP-A1- 3 057 138        WO-A1-2018/117710
US-A1- 2017 311 856     US-A1- 2017 372 152

(52) Cooperative Patent Classification (CPC): (Cont.)
A61B 5/6826; A61B 5/6831; A61B 5/6833;
A61B 5/6891; A61B 5/6892; A61B 5/6893;
A61B 5/6897; A61B 5/6898; A61B 2562/0233;
A61B 2562/185

**Description**

<u>Field</u>

The present invention relates to a photoplethysmography (PPG) sensor to be worn on a user's body for measuring a PPG signal having a high signal to noise ratio. More particularly, the invention relates to a PPG sensor comprising a sensing portion including an optical emitter and an optical detector, wherein the sensing portion is shielded from the external environment and wherein the PPG sensor can be easily manufactured with good assembly tolerances.

<u>Background</u>

According to the World Health Organization, one in three adults suffer from hypertension worldwide. Hypertension can lead to severe complications, such as stroke and heart failure. Each year, this illness results in 7.5 million premature deaths worldwide. The paradox of hypertension is that most people suffering from this condition are unaware of it.

Furthermore, the current «gold standard» for non-invasive blood pressure (BP) measurement is performed with a cuff placed around the arm. This 110 years old technology is cumbersome and leads to low compliance for patients pre-scribed to self-monitor. Consequently, healthcare professionals lack access to complete and high-quality data for their diagnosis and the treatment of this disease.

An optimal technology to monitor BP should be non-invasive (it requires no penetration into the human body), non-occlusive (it requires no pressure to be applied around a part of the human body), beat-to-beat (it allows to measure blood pressure values at each heart beat), useable any time, every day (24/7) in home environment (it allows to measure blood pressure not only in supervised clinical settings but also in real life environments out of the clinics) and comfortable (it does not generate any disturbance to its user).

In the quest for such an optimal BP technology, PPG combined with pulse wave analysis (PWA) techniques have recently arisen the interest of research institutes and the industry. While photoplethysmography (PPG) is a sensor technology that provides useable 24/7 in home environment and comfortable measurements of arterial pulsatility (the change of diameter of an artery created by the arrival of an arterial pressure pulse), PWA is a signal processing tool that allows extracting cardiovascular insights from arterial pulsatility patterns (such as heart rate and blood pressure). The combination of these two technologies is known as optical blood pressure monitoring (OBPM). See for example reference: "DOI: 10.1109/M PU L.2018.2856960" .

Among the different possible implementations of OBPM solutions, a setup is of particular interest: OBPM at the wrist. By embedding a PPG sensor into a wrist device (by instance into a bracelet, a smartband or a watch), and by applying PWA analysis techniques on the measured signals, one can build up a BP monitor that easily integrates into user's life. Such a solution opens thus the door to a first-ever cuffless and beat-to-beat monitoring of BP in ambulatory and clinical conditions, offering clinicians novel insights on BP regulation, and enabling revolutionary prevention and therapeutic strategies. See for example US20170360314A1.

The use of an OBPM solution at the wrist requires the use of a special modality of photoplethysmography, namely reflective photoplethysmography (as opposed to transmission photoplethysmography). See for example reference: ISBN-13: 978-0750304672.

A reflective PPG sensor illuminates a portion of the skin, by instance by using an off-the-shelf LED, and retrieves a portion of the light that has been propagated through the tissue, by instance by using an off-the-shelf photo-diode. Because of the presence of arterioles and other perfused structures in the skin, the amplitude of the retrieved portion of light is modulated by the blood flow and the structural changes locally occurring each time a pressure pulse reaches the illuminated portion of the skin. The analysis of the retrieved light modulations is known to be relevant to identify cardiovascular information from the user such a as heart rate, heart rate variability, blood constituents, blood pressure and other cardiovascular parameters. See for example reference: doi:10.1088/0967-3334/28/3/R01.

EP3057138 discloses a reflective optical sensor module, an optical sensing accessory, and an optical sensing device. The reflective optical sensor module comprises a light source and a first encapsulant, a photodetector and a second encapsulant, and a substrate. The light source is configured to convert electric power into radiant energy and to emit light to an object surface. The photodetector is configured to receive the light from an object surface and to convert radiant energy into electrical current or voltage. A partition is located between the light source and the photodetector. At least one medial surface of the encapsulants forms an optical directional component which may be an inclined plane or a lens. Optical directional components facilitate light extraction efficiency and light receiving efficiency, respectively.

US2017311856 discloses an apparatus and method for detecting light reflected from an object. The apparatus includes a light guiding element including a diffracting structure configured to direct incident light to an object; and an optical sensing element configured to detect light reflected from said object.

Unfortunately, current state-of-the-art PPG sensors are associated with several drawbacks. In order to shield the electrical and optoelectronics components from external aggressions (humidity, dust, ...), reflective PPG sensors integrate a "transparent layer interface" between the PPG sensor and the body. This layer generates important loses on the amount of light energy that is actually injected to and collected from the body, diminishing the overall performances of the sensor.

In order to avoid the integration of such a "transparent layer interface" some manufacturers have provided means to directly create a contact between the PPG sensor and the body. This approach is linked to safety (biocompatibility, electrical safety, ... ), usability issues (cleanliness and disinfection) and device assembly issues.

Finally, large manufacturing volumes of reflective PPG sensors (for instance for consumer-oriented products) are associated with assembly tolerance issues from the optical and mechanical elements.

Summary

The present disclosure concerns a PPG sensor according to independent claim 1.

In an embodiment the PPG sensor is configured to be worn at a wrist and for determining a blood pressure.

The PPG sensor disclosed herein has increased transmission of the emitted light resulting in a high signal to noise ratio of the PPG signal and reduced power consumption. Moreover, the sensing portion is shielded from the external environment. The PPG sensor can be easily manufactured with good assembly tolerances. These and other advantages will become apparent from the following description.

The invention is defined by the appended claims.

Brief Description of the Drawings

The invention will be better understood with the aid of the description of an embodiment given by way of example and illustrated by the figures, in which

Fig. 1 illustrates a reflective PPG comprising and optical emitter, a transparent interface element and an antireflective interface, according to an embodiment;

Fig. 2 represents transmission through the transparent interface element and an antireflective interface of the emitted light emitted by the optical emitter, according to an embodiment;

Fig. 3 illustrates the PPG sensor, wherein the optical interface does not comprise the antireflective interface;

Fig. 4 illustrates the PPG sensor, wherein the optical interface comprises the antireflective interface;

Fig. 5 shows the PPG sensor comprising a flexible interfacing structure, according to an embodiment;

Fig. 6 shows the PPG sensor with a configuration of the interfacing structure; and

Fig. 7 illustrate a PPG sensor with another configuration of the interfacing structure.

Detailed Description of possible embodiments

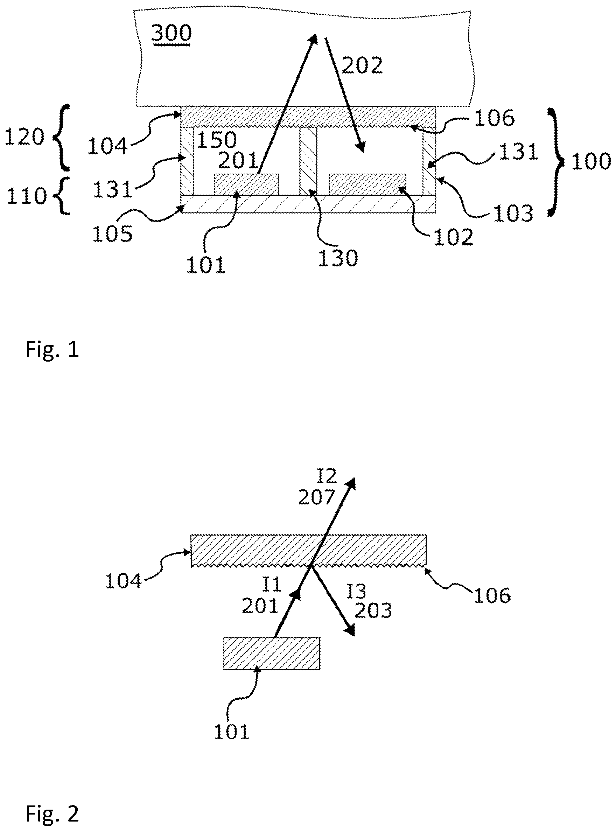

Fig. 1 illustrates a reflective PPG sensor 100 destined to be worn on a user's body 300, according to an embodiment. The PPG sensor 100 comprises a sensing portion 110 comprising an optical emitter 101 configured to emit an emitted light 201 toward a tissue of the user's body 300, and an optical detector 102 for detecting a detector light 202 reflected or scattered from the tissue, such as to provide a PPG signal. The PPG sensor 100 further comprises an interfacing portion 120 comprised between the sensing portion 110 and the user's body 300 when the PPG sensor 100 is worn. The interfacing portion 120 comprises a transparent interface element 104 through which the emitted light 201 is transmitted when travelling from the optical emitter 101 toward the tissue, and through which the detector light 202 is transmitted when travelling from the tissue to the optical detector 102.

The interfacing portion 120 comprises a gap 150 between the sensing portion 110 and the transparent interface element 104. The gap 150 is filled with a medium. Preferably, the medium is air.

In an embodiment, the transparent interface element 104 comprises an antireflective interface 106.

The transparent interface element 104 can be manufactured from a transparent material such as polycarbonate, acrylic, silicone, glass, metal oxides, ceramics and any other suitable transparent material with low absorption coefficient in the emitted wavelength and refractive index close to human skin refractive index. The low absorption coefficient can be in the green wavelength range (between 500 and 575 nm), in the infra-red wavelength range (above 750 nm), in the red wavelength range (between 650 and 750 nm), in the yellow wavelength (between 575 and 650)), in the blue wavelength (420 and 500 nm) or any other wavelength that can penetrate the user's body 300 (between 400 and 1500 nm). The transparent material can be biocompatible with human or animal tissues (skin). The transparent interface element 104, (with or without the antireflective interface 106) can be configured to exhibit light transmission of greater than 70% or 80%, but preferably greater than 90%.

The antireflective interface 106 can comprise any suitable antireflective means or antireflective coating. For example, the antireflective interface 106 can comprise any one, alone or in combination, of an index-matching coating, a single-layer interferential coating, a multi-layer interferential coating, a texturized layer, or any other type of antireflective coating.

A represented in Fig. 2, the emitted light 201 is emitted by the optical emitter 101 with a emitted intensity 11. The emitted light 201 is incident on the surface of the transparent interface element 104. A reflected portion 203 of the emitted light 201 of reflected intensity I3 is reflected, and a transmitted portion 207 of the emitted light 201 with a transmitted intensity I2 is transmitted through the transparent interface element 104 (into the user's body 300).

When the emitted light 201 meets the interface of the transparent interface element 104 at normal incidence (perpendicularly to the surface), the reflected intensity I3 of light reflected is given by the reflection coefficient, or reflectance R:

$$R = \left(\frac{n_2 - n_1}{n_2 + n_1}\right)^2 \tag{1}.$$

where $n_1$ and $n_2$ are the refractive indexes of the first and second media respectively, i.e., of the transparent interface element 104 and the gap 150.

If absorption is neglected, the transmittance (or transmission coefficient) T corresponds to 1 - R. In the case of the emitted light 201 travelling from the gap 150 being filled with air and having a second refractive index $n_2 \approx 1.0$, into the transparent interface element 104 having a first refractive index $n_1 \approx 1.5$, the value of R is 0.04, or 4%, on a single reflection. So, the transparent interface element 104 has an interface transmittance T1 of 96%, i.e., at most 96% of the light (T = 1 - R = 0.96) actually enters the transparent interface element 104, and the rest is reflected from its surface.

The interface transmittance T1 corresponds to the ratio of the transmitted intensity I2 on the emitted intensity I1:

$$T1 = {I2}/{I1} = 96\% \tag{2}.$$

In other words, if the emitted light 201 with emitted intensity I1 is incident on the surface of the transparent interface element 104 (without the antireflective interface 106), the reflected portion 203 of reflected intensity I3 is reflected, and the transmitted portion 207 with transmitted intensity I2 is transmitted into the user's body 300.

In the case the transparent interface element 104 comprises the antireflective interface 106, the latter can be configured such that an antireflective interface transmittance T2, i.e., the ratio of the transmitted intensity I2 on the emitted intensity I1, is at least 99%:

$$T2 = {I2}/{I1} = 99\% \tag{3}.$$

Although not shown in Fig. 2, the detector light 202 can also be reflected at the surface of the transparent interface element 104 comprising the antireflective interface 106, such that the antireflective interface transmittance of the transmitted portion of the detector light 202 will also be higher than without the antireflective interface 106.

More generally, the antireflective interface 106 can be configured such that the transparent interface element 104 comprising the antireflective interface 106 has an antireflective interface transmittance T2 of at least 99%.

In an embodiment, the antireflective interface 106 is configured such that, for the transparent interface element 104 having the first refractive index $n_1$ being larger than the second refractive index $n_2$ of the gap 150, the transparent interface element 104 comprising the antireflective interface 106 has an antireflective interface transmittance T2 of at least 99%.

In other words, the antireflective interface 106 is configured such that the antireflective interface transmittance T2 of the transparent interface element 104 comprising the antireflective interface 106 is larger than the interface transmittance T1 of the transparent interface element 104 by at least 4%, or:

$$T2 \geq 1.04\, T1 \tag{4}$$

The antireflective interface 106 is configured such that the antireflective interface transmittance T2 of at least 99% for the second refractive index $n_2$ being larger than the first refractive index $n_1$ by at least 10%, for example about 20% or about 30%. The second refractive index $n_2$ depends of the medium filling the gap 150. As mentioned, the medium can comprise air and any other low refractive index material such as highly porous specular $SiO_2$, having a refractive index value as low as 1.05, or low refractive index polymers, having a refractive index value for example between 1.2 and 1.4.

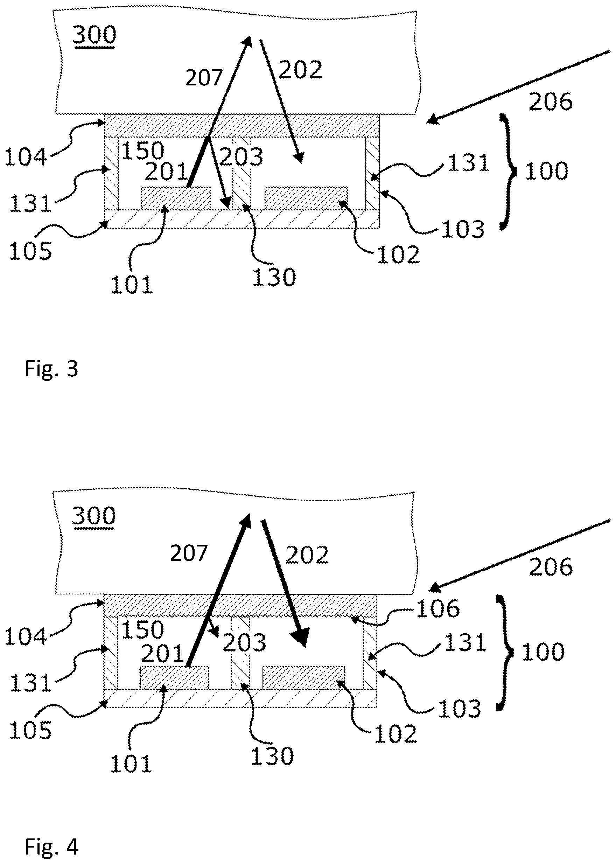

Fig. 3 illustrates the PPG sensor 100, wherein the optical interface 104 does not comprise the antireflective interface 106. In this configuration, a reflected portion 203 of the emitted light 201 is reflected on transparent interface element 104 back toward the sensing portion 110, reducing the transmitted intensity I2 of the transmitted portion 207 of emitted light 201 reaching the user's body 300 as well as the intensity of the detector light 202 reaching the optical detector 102. Although Fig. 3 represents the reflected portion 203 of the emitted light 201 as a single reflection path, several reflection paths and reflection angles are possible.

Fig. 4 illustrates how the addition of the antireflective interface 106 to the optical interface 104 reduces the reflected portion 203 of the emitted light 201 and increases the transmitted intensity I2 of transmitted portion 207 of emitted light 201 reaching the user body 300 and the intensity of the detector light 202 reaching the optical detector 102.

The increased transmission of the emitted light 201 results in an increased signal to noise ratio of the PPG signal. The PPG sensor 100 can thus have a reduced power consumption (thus longer battery life) by reducing the intensity of the emitted light 201 and obtaining a similar amplitude of the measured PPG signal.

For the same reasons as above, the transparent interface element 104 comprising the antireflective interface 106 has also an increased antireflective interface transmittance T2 for the detector light 202 coming from the user's body 300 and travelling toward the optical detector 102. Due to the increased antireflective interface transmittance T2 for both the emitted light 201 and the detector light 202 when passing through the transparent interface element 104 comprising the antireflective interface 106, the emitted intensity I1 of the emitted light 201 generated by the optical emitter 101 and the intensity of the detector light can be lower than what is possible with a conventional PPG sensor for obtaining the same signal to noise ratio of the PPG signal.

As shown in the example of Fig. 1, the sensing portion 110 comprises a base 105 on which the optical emitter 101 and the optical detector 102 are provided. The base 105 can comprise a circuit board, a PCB, an electronic support, an adhesive patch, a textile, the skin itself or any other support. The base 105 can be configured as to be flexible or rigid or in part flexible and rigid. The base 105 can be configured to support or/and be connected to various electronic components such as an analog front end (AFE), a microcontroller (MCU), a central processing unit (CPU), a memory, a wireless module, a power source, and/or any other electrical component.

In a possible configuration, the sensing portion 110 can be integrated in a single chip, such as the Biofy® sensor manufactured by OSRAM. The sensing portion 110 can be provided as a complete module with additional features and / or processing, such as the products MAX30100/1/2 or MAX86160 manufactured by Maxim Integrated, ADPD142/4RI, ADPD174/5GGI or ADPD188BI manufactured by Analog Devices, BW1/2/4 or BE2/4 manufactured by Valencell, AS7000 or AS7024 manufactured by AMS, and Si1143/4 or Si1172/3 manufactured by SiLabs. base 105, the optical emitter 101 and the optical detector 102 can also be provided or be mounted as separate components.

The optical emitter 101 can comprise a single LED (as the SFH 4043, LT PWSG, CT DELSS1.12 or CH DELSS 1.22 by OSRAM, or multiple LEDs (for example the SFH 7013 from OSRAM, VSMD66694 by Vishay and DLED-xxx/xxx-CSL-2/3 by OSI optoelectronics), or any other optical source such as diodes, organic diodes, polymer diodes, laser diodes, or all other sources alike. The wavelength of the emitted light by the optical emitter 101 can be in the green wavelength range (between 500 and 575 nm), in the infra-red wave-length range (above 750 nm), in the red wavelength range (between 650 and 750 nm), in the yellow wavelength (between 575 and 650)), in the blue wavelength (420 and 500 nm) or any other wavelength that can penetrate the body of a user (between 400 and 1500nm).

The optical detector 102 can comprise a photo-diode, a photodetector, a phototransistor, an optical chipset, a camera imaging device, a charge-coupled device or all other receivers alike (for example the D5140 SFH 2200, SFH 2440, BPW 34 5, SFH2201 or SFH 2704 by OSRAM, VCNL3036 or VEMD5010X01 by Vishay, BH179XGLC by ROHM, ADPD221X by Analog Devices, Si1181/2 by SiLabs and PIN-0.81-LLS, PIN-4.0-LLS or PIN-8.0-LLS from OSI optoelectronics). The optical detector 102 can be able to collect light at a large optical spectrum, or at a notched spectrum window that matches the emission wavelength of the optical emitter 101.

The optical emitter 101 and the optical detector 102 can be separated at different distances among each other (1.33, 1.45, 2.18, 2.12, 2.32, 2.4, 2.51, 2.71, 2.72, 2.8, 2.83, 2.94, 2.95, 3.11, 3.5 and 5.3 mm) and can be configured to be at different distances from the user's body 300 (in direct contact with the body or contactless). It is known that different distances between the optical emitter 101 and the optical detector 102 and different distances from the body 300 lead to different penetration depths of the emitted light 201 and the detector light 202 reflected or scattered into the body 300.

In other variants, the transparent interface element 104 comprising the antireflective interface 106 can be flat or have a non-null radius of curvature. For example, the transparent interface element 104 comprising the antireflective interface 106 can have radius of curvature between 10 and 100 mm.

The shape of the transparent interface element 104 comprising the antireflective interface 106 can be configured such as to optimize the contact pressure with the user's body 300 when the PPG sensor 100 is worn, in order to reduce possible movements between the PPG sensor 100 and the body 300. The shape and roughness of the transparent interface element 104 comprising the antireflective interface 106 can further be configured to optimize the contact between the transparent interface element 104 and the body 300 in order maximize the transmitted intensity I2 of the transmitted portion 207 of emitted light 201 and the intensity of the detector light 202.

According to an embodiment, the antireflective interface 106 comprises a textured surface. Preferably, the antireflective interface 106 comprises a textured surface having a roughness Ra between 0.025 and 0.40 $\mu$m. Such roughness corresponds to the SPI finishes A-2 to C-2, according to SPI Mold Surface Finish Standards, or to VDI Standards. The textured surface can be produced by molding, forming, machining or any other suitable technique.

In the configuration shown in Fig. 1, the antireflective interface 106 is comprised on one side of the transparent interface element 104. In the specific example of Fig. 1, the antireflective interface 106 is comprised on the side of the transparent interface element 104 that is closer to the sensing portion 110. Moreover, in the specific example of Fig. 1 the side of the transparent interface element 104 devoid of the antireflective interface 106 can be in direct contact with a tissue (such as the skin) of the user's body 300. The latter configuration allows for increasing the transmission of the emitted light 201 by reducing the reflected portion of emitted light 203 caused by the refraction index change of the air and the transparent interface element 104 comprising the antireflective interface 106 (i.e., the surface of the transparent interface element 104 farther from the sensing portion 110).

Other arrangements (not illustrated) of the interfacing portion 120 are however contemplated, as far as they fall under the scope of the appended claims. For example, the antireflective interface 106 may be comprised on the side of the transparent interface element 104 that is farther to the sensing portion 110, and thus, destined to be closer to the user's body 300 when the PPG sensor 100 is worn. Such a configuration allows for increasing further the antireflective interface transmittance T2 of the transparent interface element 104.

In another possible arrangement, the antireflective interface 106 can be in direct contact with a tissue (such as the skin) of the user's body 300. In yet another possible arrangement, the interfacing portion 120 can be configured such that the antireflective interface 106 does not come into contact with the user's body 300 when the PPG sensor is worn.

In yet another arrangement, the antireflective interface 106 can be comprised on both sides of the transparent interface element 104. This configuration allows for increasing the transmission of the emitted light 201 by reducing the reflected portion of emitted light 203 caused by the refraction index change of the air and the transparent interface element 104 comprising the antireflective interface 106. The antireflective interface 106 on the side of the transparent interface element 104 farther from the sensing portion 110 increases the transmission of the emitted light 201 by reducing the reflected portion of emitted light 203 caused by the change in refraction index of the tissue of the user's body 300 and the transparent interface element 104 comprising the antireflective interface 106.

The PPG sensor 100, can comprise a contact PPG sensor, wherein the transparent interface element 104, comprising the antireflective interface 106 on any one of the sides of the transparent interface element 104 or on both sides of the transparent interface element 104, is in contact (possibly in direct contact) with the user's body 300. Alternatively, the PPG sensor 100, can comprise a non-contact PPG sensor, wherein the transparent interface element 104, comprising the antireflective interface 106 on any one of the sides of the transparent interface element 104 or on both sides of the transparent interface element 104, is not in contact with the user's body 300. Example of a non-contact PPG sensor 100 can include an emitting laser as a light source and a high-speed PiN photodiode as a photodetector (see for example reference: oai:dspace.lboro.ac.uk:2134/22188).

In an embodiment, the interfacing portion 120 comprises an interfacing structure 103, extending between the base 105 and the transparent interface element 104 such as to define the gap 150 between the sensing portion 110 and the transparent interface element 104. In the example shown in Figs. 1-3, the interfacing structure 103 comprises a middle wall 130, between the optical emitter 101 and the optical detector 102, and lateral walls 131 surrounding the optical emitter 101 and optical detector 102.

The interfacing structure 103 can be configured such that the gap 150 is completely enclosed between the base 105, the walls 130, 131 and the transparent interface element 104. The interfacing structure 103 can be configured such that the gap 150 is hermetically sealed from the exterior environment. In other variants, the interfacing structure 103 can be configured such that the gap 150 is open to the exterior environment. Here, the middle wall 130 and lateral walls 131 can comprise opening or can comprise pillars or any other open structure.

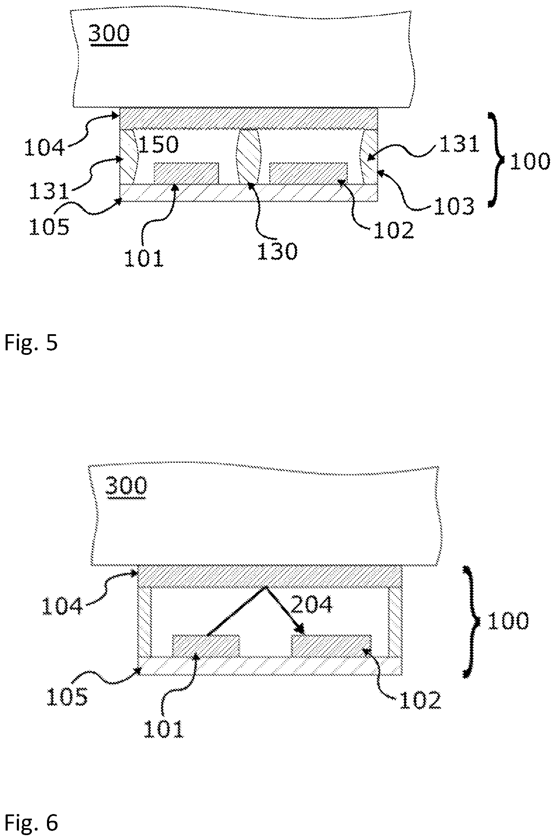

In a preferred embodiment, the interfacing structure 103 is flexible such that the distance between the base 105 and the transparent interface element 104, and this the height (or volume) of the gap 150 can be varied when a pressure is applied either on the base 105 or the transparent interface element 104 or on both (see Fig. 5).

The flexible interfacing structure 103 further allows for compensating assembly tolerances of the sensing portion 110 by correcting the assembly imperfections of the different optical and mechanical elements forming the sensing portion 110.

The flexible interfacing structure 103 facilitates the integration of commercially-available sensing portion 110 (such as the Biofy® sensor and other commercially-available sensors described above) with a great flexibility in design. The manufacturing of the PPG sensor 100 is thus facilitated.

The transparent interface element 104 in combination with the interfacing structure 103 allows for shielding the sensing portion 110 from the exterior environment (such as protecting the sensing portion 110 from dust, water and moisture). The transparent interface element 104, possibly comprising the antireflective interface 106, can be cleaned. The transparent interface element 104 further allows for insulating the sensing portion 110 from the tissue of the user's body 300 and provide biocompatibility when the PPG sensor 100 is in contact with the tissue of the user's body 300.

In known PPG sensor configurations, the optical emitter 101 and the optical detector 102 are in direct contact with the user's body 300 (such as skin) or are in contact via a medium material, wherein the medium material can be the transparent interface element 104 or any other material have a refractive index that is close to the refractive index of the user's body 300 (typically around 1.4 - 1.5).

In contrast, the PPG sensor 100 described herein comprises the gap 150 between the optical emitter 101 and the optical detector 102 and the transparent interface element 104. As mentioned above, the gap 150 can be filled with air or another medium. In the case of air, and possibly for another medium, the refractive index can be close to 1 resulting in a large reflected portion 203 of emitted light 201 at the interface between the gap 150 and the transparent interface element 104 and thus a low interface transmittance T1.

The transparent interface element 104 comprising the antireflective interface 106 allows for obtaining a ratio of the transmitted intensity I2 on the emitted intensity I1 of or larger than 99%, in the case of the gap 150 having a refractive index can be close to 1 being comprised between the optical emitter 101 and the optical detector 102 and the transparent interface element 104. In other words, the antireflective interface 106 can be configured such that the antireflective interface transmittance T2 of the transparent interface element 104 comprising the antireflective interface 106 is larger than the interface transmittance T1 of the transparent interface element 104 (without the antireflective interface 106) by at least 4%.

The interfacing structure 103 can be further configured to prevent optical shunting, i.e. to prevent the emitted light 201 to reach the optical detector 102 without travelling through the transparent interface element 104 and the tissue of the user's body 300, and/or prevent ambient light 206 reaching the optical detector 102. In other words, the interfacing structure 103 can be used as an optical mask. Here, ambient light 206 is the light coming from the environment, external to the PPG sensor 100.

As shown in Figs. 1 to 3, the middle wall 130 optically shields the optical emitter 101 from the optical detector 102, preventing the emitted light 201 to reach the optical detector 102 without travelling through the transparent interface element 104 and the tissue of the user's body 300. The lateral walls 131 can be configured to prevent ambient light 206 to reach the optical detector 102.

To that end, the interfacing structure 103 can be made from a light blocking or/and light reflective material. The interfacing structure 103 can comprise a color or/and surface finish being a dark or bright. The interfacing structure 103 can be made from a material that does not absorbs light.

Fig. 6 shows a variant of the PPG sensor 100, wherein the interfacing structure 103 does not comprise the middle wall and wherein a shunted portion 204 of emitted light 201 travels towards the optical detector 102 without passing through the transparent interface element 104 and the user's body 300.

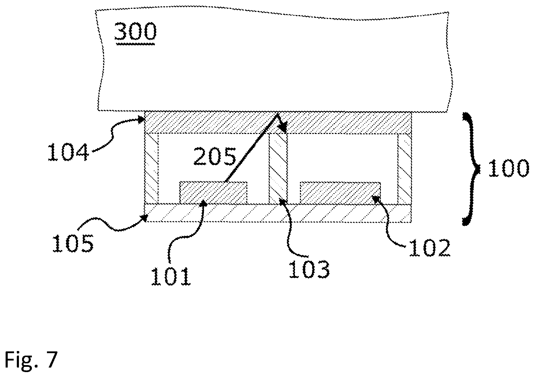

Fig. 7 illustrates an optimal dimensioning of the interfacing structure 103, wherein the middle wall 130 extends between the base 105 and the transparent interface element 104 and reduces the interface reflected portion 205 by preventing it from travelling from the optical detector 102 to the optical detector 102 without penetrating in the user's body 300. The dimensioning of the interfacing structure 103 is also configured to prevent emitted light 201 reflecting at the surface of the transparent interface element 104 farther from the sensing portion 110 and not travelling further, i.e., not penetrating into the user's body 300. The dimensioning of the interfacing structure 103 is based on reflection and refraction angles of the emitted light 201 and detector light 202.

The PPG sensor 100 having improved optical performances allows to explore novel configurations adapted to different applications. For instance, the PPG sensor 100 can be a small-sized (such as a PPG sensor 100 having the optical emitter 101 and the optical detector 102 spatially separated by 4 to 10 mm), or large-sized (such as a PPG sensor 100 having the optical emitter 101 and the optical detector 102 separated by more than 10 mm). Alternatively or in combination, the wavelength or wavelength range of the optical emitter 101 can be selected, as well as filtering configurations of the optical detector 102 in order to allow either superficial or deep penetration depths of the emitted light 201 in the tissue of the user's body 300 and measuring arterial pulsatilities for different layers of the user's body 300 tissue.

The PPG sensor 100 can be configured to be worn at a location of the user's body 300, including any one of: an armband, a wrist, a fingertip, or an ear.

The PPG sensor 100 can be configured to be integrated in wearable device including any one of: a headband, an ankle bracelet, a choker, a ring, a helmet, an ear plug, a hearing aid, a headphone, glasses, a shirt, a bra, a garment, a glove, underpants, a socket, a shoe, a wearable sensor, a patch adhering to the skin of the user, a pulsatility signal sensor.

The PPG sensor 100 can be further configured such as to be integrated in a device destined to be in contact with the user's body 300, including any one of: a bed sensor, a chair sensor, a toilet sensor, a table sensor, a car sensor, a computer mouse sensor, or any other arrangement destined to measure a pulsatility signal.

Due to the increased transmission of the emitted light 201 and the increased signal to noise ratio of the PPG signal, the PPG sensor 100 facilitates the measurement of local arterial pulsatility. Indeed, an increased signal to noise ratio of the PPG signal improves the performances of the signal processing algorithms, such as analysis of time series of local arterial pulsatility, used for determining cardiovascular parameters such as blood pressure.

The PPG sensor 100 can be used for determining blood pressure, pulse pressure, central pulse wave velocity, peripheral pulse wave velocity, arterial stiffness, aortic pulse transit time, augmentation index, stroke volume, stroke volume variations, pulse pressure variations, cardiac output, systemic vascular resistance, venous pressure, systemic hemodynamic parameters, pulmonary hemodynamic parameters, cerebral hemodynamic parameters, heart rate, heart rate variability, inter-beat intervals, arrhythmias detection, ejection duration, SpO2, SpHb, SpMet, SpCO, respiratory rate, tidal volume, apnea detection, sleep quality, sleep scoring, sleep analysis, bed time, sleep duration, rem sleep time, light sleep time, deep sleep time, time to get up, time to sleep, sleep efficiency, minutes awake after sleep onset, snoring duration, stress indexes, and general cardiovascular and health indexes.

The invention is defined by the appended claims.

Reference numbers

[0073]

| | |
|---|---|
| 100 | PPG sensor |
| 101 | optical emitter |
| 102 | optical detector |
| 103 | interfacing structure |
| 104 | transparent interface element |
| 105 | base |
| 106 | antireflective interface |
| 110 | sensing portion |
| 120 | interfacing portion |
| 130 | middle wall |
| 131 | lateral wall |
| 150 | gap |
| 201 | emitted light |
| 202 | detector light |
| 203 | reflected portion of emitted light |
| 204 | shunted portion of emitted light |
| 205 | interface reflected portion of emitted light |
| 206 | ambient light |
| 207 | transmitted portion of emitted light |
| 300 | user body |
| I1 | emitted intensity |
| I2 | transmitted intensity |
| I3 | reflected intensity |
| $n, n_1, n_2$ | refractive index |
| T1 | interface transmittance |
| T2 | antireflective interface transmittance |

**Claims**

1. A photoplethysmography (PPG) sensor (100) configured to be worn on or to be in contact with a user's body (300), the PPG sensor (100) comprising:

a sensing portion (110) comprising an optical emitter (101) configured to emit an emitted light (201) toward the user's body (300), and an optical detector (102) for detecting a detector light (202) reflected or scattered from the tissue, such as to provide a PPG signal; and

an interfacing portion (120) comprised between the sensing portion (110) and the user's body (300), the interfacing portion (120) comprising a transparent interface element (104) through which the emitted light (201) is transmitted when travelling from the optical emitter (101) toward the body (300), and through which the detector light (202) is transmitted when travelling from the body (300) to the optical detector (102), wherein the transparent interface element (104) has an interface transmittance (T1);

the interfacing portion (120) comprises a gap (150) between the sensing portion (110) and the transparent interface element (104), the transparent interface element (104) having a first refractive index ($n_1$) and the gap (150) having a second refractive index ($n_2$);

**characterized in that**

the transparent interface element (104) comprises an antireflective interface (106) being configured such that, when the second refractive index (nz) is larger than the first refractive index ($n_1$) by at least 10%, an antireflective interface transmittance (T2) of the transparent interface element (104) comprising the antireflective interface (106) is at least 99%.

2. The PPG sensor according to claim 1,
   wherein the second refractive index ($n_2$) is larger than the first refractive index ($n_1$) by about 20% or about 30%.

3. The PPG sensor according to claim 1 or 2,
   wherein the antireflective interface (106) comprises a textured surface having a roughness Ra between 0.025 and 0.40 $\mu$m.

4. The PPG sensor according to any one of claims 1 to 3,
   wherein the antireflective interface (106) comprises any one of an index-matching coating, a single-layer interference coating, a multi-layer interference coating, or any other type of antireflective coating.

5. The PPG sensor according to any one of claims 1 to 4,
   wherein the antireflective interface (106) is comprised on one of the sides of the transparent interface element (104).

6. The PPG sensor according to claim 5,
   wherein the antireflective interface (106) is comprised on the side of the transparent interface element (104) that is closer to the sensing portion.

7. The PPG sensor according to claim 5,
   wherein the antireflective interface (106) is comprised on the side of the transparent interface element (104) that is farther to the sensing portion.

8. The PPG sensor according to any one of claims 1 to 4,
   wherein the antireflective interface (106) is comprised on both sides of the transparent interface element (104).

9. The PPG sensor according to any one of claims 1 to 8, comprising a contact PPG sensor (100) or a non-contact PPG sensor (100).

10. The PPG sensor according to any one of claims 1 to 9,

    wherein the sensing portion comprises a base (105) on which the optical emitter (101) and the optical detector (102) are provided, and
    wherein the interfacing portion comprises an interfacing structure (103) extending between the base plate (105) and the transparent interface element (104), such as to define the gap (150) between the sensing portion (110) and the transparent interface element (104).

11. The PPG sensor according to claim 10,
    wherein the interfacing structure (103) comprises a middle wall (130), between the optical emitter (101) and the optical detector (102), and lateral walls (131) surrounding the optical emitter (101) and optical detector (102).

12. The PPG sensor according to claim 10 or 11,

wherein the interfacing structure (103) is configured such that the gap (150) is hermetically sealed from the exterior environment.

13. The PPG sensor according to any one of claims 10 to 12,
wherein the interfacing structure (103) is flexible such that the distance between the base (105) and the transparent interface element (104) can be varied when a pressure is applied either on the base (105) or the transparent interface element (104) or on both.

14. The PPG sensor according to any one of claims 10 to 13,
wherein the interfacing structure (103) is further configured to prevent the emitted light (201) to reach the optical detector (102) without travelling through the transparent interface element (104) and the user's body (300).

15. The PPG sensor according to claim 14,
wherein the interfacing structure (103) is further configured to prevent ambient light (206) from reaching the optical detector (102).

16. The PPG sensor according to any one of claims 1 to 15, configured to be worn on a user's body (300) location including any one of:

an armband, a wrist, a fingertip, or an ear; or
configured to be integrated in wearable device including any one of: a headband, an ankle bracelet, a choker, a ring, a helmet, an ear plug, a hearing aid, a headphone, glasses, a shirt, a bra, a garment, a glove, underpants, a socket, a shoe, a wearable sensor, a patch adhering to the skin of the user, a pulsatility signal sensor; or
configured to be integrated in a device destined to be in contact with the user's body (300), including any one of: a bed sensor, a chair sensor, a toilet sensor, a table sensor, a car sensor, a computer mouse sensor.

17. The PPG sensor according to any one of claims 1 to 16, configured to be worn at a wrist and for determining a blood pressure from the PPG signal.

**Patentansprüche**

1. Ein Photoplethysmographie-(PPG-)Sensor (100), welcher zum Tragen am Körper (300) eines Benutzers konfiguriert ist, wobei der PPG-Sensor (100) Folgendes umfasst:

einen Sensorabschnitt (110) mit einem optischen Emitter (101), welcher so konfiguriert ist, dass er ein emittiertes Licht (201) in Richtung des Körpers (300) des Benutzers aussendet, und einem optischen Detektor (102) zum Erfassen eines vom Gewebe reflektierten oder gestreuten Detektorlichts (202), um so ein PPG-Signal bereitzustellen; und
einen Schnittstellenabschnitt (120), welcher zwischen dem Sensorabschnitt (110) und dem Körper (300) des Benutzers angeordnet ist, wobei der Schnittstellenabschnitt (120) ein transparentes Schnittstellenelement (104) umfasst, durch welches das emittierte Licht (201) übertragen wird, wenn es sich vom optischen Emitter (101) in Richtung des Körpers (300) bewegt, und durch welchen das Detektorlicht (202) übertragen wird, wenn es sich vom Körper (300) zum optischen Detektor (102) bewegt, worin das transparente Schnittstellenelement (104) eine Schnittstellendurchlässigkeit (T1) aufweist;
wobei der Schnittstellenabschnitt (120) einen Spalt (150) zwischen dem Sensorabschnitt (110) und dem transparenten Schnittstellenelement (104) umfasst, und das transparente Schnittstellenelement (104) einen ersten Brechungsindex ($n_1$) und der Spalt (150) einen zweiten Brechungsindex ($n_2$) aufweist
**dadurch gekennzeichnet, dass**
das transparente Schnittstellenelement (104) eine Antireflex-Schnittstelle (106) umfasst, welche derart konfiguriert ist, dass wenn der zweite Brechungsindex ($n_2$) grösser als der erste Brechungsindex ($n_1$) um mindestens 10%, eine Antireflex-Schnittstellendurchlässigkeit (T2) des transparenten Schnittstellenelements (104) mit der Antireflex-Schnittstelle (106) bei mindestens 99% liegt.

2. PPG-Sensor gemäss Anspruch 1,
worin der zweite Brechungsindex ($n_2$) um etwa 20 % oder etwa 30 % grösser als der erste Brechungsindex ($n_1$) ist.

3. PPG-Sensor gemäss einem Anspruch 1 oder 2, worin die Antireflex-Schnittstelle (106) eine strukturierte Oberfläche mit einer Rauheit Ra zwischen 0,025 und 0,40 μm aufweist.

4. PPG-Sensor gemäss irgendeinem der Ansprüche 1 bis 3, worin die Antireflex-Schnittstelle (106) irgendeine der Folgenden umfasst: eine Indexanpassungsbeschichtung, eine einschichtige Interferenzbeschichtung, eine mehrschichtige Interferenzbeschichtung oder eine beliebige andere Art von Antireflex-Beschichtung.

5. PPG-Sensor gemäss irgendeinem der Ansprüche 1 bis 4, worin die Antireflex-Schnittstelle (106) auf einer der Seiten des transparenten Schnittstellenelements (104) angeordnet ist.

6. PPG-Sensor gemäss Anspruch 5, worin die Antireflex-Schnittstelle (106) auf der Seite des transparenten Schnittstellenelements (104) angeordnet ist, welche näher am Sensorabschnitt liegt.

7. PPG-Sensor gemäss Anspruch 5, worin die Antireflex-Schnittstelle (106) auf der Seite des transparenten Schnittstellenelements (104) angeordnet ist, welche vom Sensorabschnitt weiter entfernt ist.

8. PPG-Sensor gemäss irgendeinem der Ansprüche 1 bis 4, worin die Antireflex-Schnittstelle (106) auf beiden Seiten des transparenten Schnittstellenelements (104) angeordnet ist.

9. PPG-Sensor gemäss irgendeinem der Ansprüche 1 bis 8, welcher einen Kontakt-PPG-Sensor (100) oder einen berührungslosen PPG-Sensor (100) umfasst.

10. PPG-Sensor gemäss irgendeinem der Ansprüche 1 bis 9, worin der Sensorabschnitt eine Basis (105) umfasst, auf welcher der optische Emitter (101) und der optische Detektor (102) vorgesehen sind, und
worin der Schnittstellenabschnitt eine Schnittstellenstruktur (103) umfasst, welche sich zwischen der Grundplatte (105) und dem transparenten Schnittstellenelement (104) erstreckt, um so den Spalt (150) zwischen dem Sensorabschnitt (110) und dem transparenten Schnittstellenelement (104) zu definieren.

11. PPG-Sensor gemäss Anspruch 10, worin die Schnittstellenstruktur (103) eine Mittelwand (130) zwischen dem optischen Emitter (101) und dem optischen Detektor (102) sowie Seitenwände (131) umfasst, welche den optischen Emitter (101) und den optischen Detektor (102) umgeben.

12. PPG-Sensor gemäss Anspruch 10 oder 11, worin die Schnittstellenstruktur (103) derart konfiguriert ist, dass der Spalt (150) hermetisch von der Aussenumgebung abgedichtet ist.

13. PPG-Sensor gemäss irgendeinem der Ansprüche 10 bis 12, worin die Schnittstellenstruktur (103) flexibel ist, so dass der Abstand zwischen der Basis (105) und dem transparenten Schnittstellenelement (104) variiert werden kann, wenn einen Druck entweder auf die Basis (105) oder das transparentes Schnittstellenelement (104) oder auf beiden ausgeübt wird.

14. PPG-Sensor gemäss irgendeinem der Ansprüche 10 bis 13, worin die Schnittstellenstruktur (103) ferner dazu konfiguriert ist, um zu verhindern, dass das emittierte Licht (201) den optischen Detektor (102) erreicht, ohne durch das transparente Schnittstellenelement (104) und den Körper des Benutzers (300) zu durchlaufen.

15. PPG-Sensor gemäss Anspruch 14, worin die Schnittstellenstruktur (103) ferner dazu konfiguriert ist, um zu verhindern, dass Umgebungslicht (206) den optischen Detektor (102) erreicht.

16. PPG-Sensor gemäss irgendeinem der Ansprüche 1 bis 15,

derart konfiguriert, dass er an einer der folgenden Stellen am Körper (300) eines Benutzers getragen werden kann: an einer Armbinde, am Handgelenk, an einer Fingerspitze oder an einem Ohr; oder
konfiguriert für die Integration in ein tragbares Gerät, darunter eines der Folgenden: ein Stirnband, eine Fussfessel, ein Halsband, ein Ring, ein Helm, ein Ohrstöpsel, ein Hörgerät, ein Kopfhörer, eine Brille, ein Hemd, ein Büstenhalter, ein Kleidungsstück, ein Handschuh, eine Unterhose, eine Socke, ein Schuh, ein tragbarer Sensor, ein auf der Haut des Benutzers haftendes Pflaster oder ein Pulsatilitätssignalsensor; oder
konfiguriert für die Integration in ein Gerät, welches dazu bestimmt ist, mit dem Körper (300) des Benutzers in Kontakt zu kommen, darunter eines der Folgenden: ein Bettsensor, ein Stuhlsensor, ein Toilettensensor, ein Tischsensor, ein Autosensor oder ein Computermaussensor.

**17.** PPG-Sensor gemäss einem der Ansprüche 1 bis 16, konfiguriert zum Tragen am Handgelenk und zur Ermittlung des Blutdrucks aus dem PPG-Signal.

**Revendications**

**1.** Capteur de photopléthysmographie (PPG) (100) configuré pour être porté sur ou en contact avec le corps d'un utilisateur (300), le capteur PPG (100) comprenant :

une partie de détection (110) comprenant un émetteur optique (101) configuré pour émettre une lumière émise (201) vers le corps de l'utilisateur (300), et un détecteur optique (102) pour détecter une lumière de détecteur (202) réfléchie ou diffusée depuis le tissu, de manière à fournir un signal PPG ; et
une partie d'interface (120) comprise entre la partie de détection (110) et le corps de l'utilisateur (300), la partie d'interface (120) comprenant un élément d'interface transparent (104) à travers lequel la lumière émise (201) est transmise lors du déplacement depuis l'émetteur optique (101) vers le corps (300), et à travers lequel la lumière du détecteur (202) est transmise lorsqu'elle se déplace depuis le corps (300) vers le détecteur optique (102), dans lequel l'élément d'interface transparent (104) a une transmission d'interface (T1) ;
la partie d'interface (120) comprend un espace (150) entre la partie de détection (110) et l'élément d'interface transparent (104), l'élément d'interface transparent (104) ayant un premier indice de réfraction ($n_1$) et l'espace (150) ayant un deuxième indice de réfraction ($n_2$) ;
**caractérisé en ce que**
l'élément d'interface transparent (104) comprend une interface anti-réfléchissante (106), qui est configurée de telle sorte que, lorsque le deuxième indice de réfraction ($n_2$) soit supérieur au premier indice de réfraction ($n_1$) d'au moins 10 %, une transmission d'interface anti-réfléchissante (T2) de l'élément d'interface transparent (104) comprenant l'interface anti-réfléchissante (106) est au moins 99%.

**2.** Capteur PPG selon la revendication 1, dans lequel le deuxième indice de réfraction ($n_2$) est supérieur au premier indice de réfraction ($n_1$) d'environ 20 % ou d'environ 30 %.

**3.** Capteur PPG selon l'une des revendications 1 ou 2, dans lequel l'interface anti-réfléchissante (106) comprend une surface texturée ayant une rugosité Ra comprise entre 0,025 et 0,40 $\mu$m.

**4.** Capteur PPG selon l'une quelconque des revendications 1 à 3, dans lequel l'interface anti-réfléchissante (106) comprend l'un quelconque parmi un revêtement d'adaptation d'indice, un revêtement interférentiel monocouche, un revêtement interférentiel multicouche, ou tout autre type de revêtement antireflet.

**5.** Capteur PPG selon l'une quelconque des revendications 1 à 4, dans lequel l'interface anti-réfléchissante (106) est comprise sur l'un des côtés de l'élément d'interface transparent (104).

**6.** Capteur PPG selon la revendication 5, dans lequel l'interface antireflet (106) est comprise sur le côté de l'élément d'interface transparent (104) qui est le plus proche de la partie de détection.

**7.** Capteur PPG selon la revendication 5, dans lequel l'interface anti-réfléchissante (106) est située sur le côté de l'élément d'interface transparent (104) qui est le plus éloigné de la partie de détection.

**8.** Capteur PPG selon l'une quelconque des revendications 1 à 4, dans lequel l'interface anti-réfléchissante (106) est comprise sur les deux côtés de l'élément d'interface transparent (104).

**9.** Capteur PPG selon l'une quelconque des revendications 1 à 8, comprenant un capteur PPG à contact (100) ou un capteur PPG sans contact (100).

**10.** Capteur PPG selon l'une quelconque des revendications 1 à 9, dans lequel la partie de détection comprend une base (105) sur laquelle l'émetteur optique (101) et le détecteur optique (102) sont prévus, et
dans lequel la partie d'interface comprend une structure d'interface (103) s'étendant entre la plaque de base (105) et l'élément d'interface transparent (104), de manière à définir l'espace (150) entre la partie de détection (110) et l'élément d'interface transparent (104).

**11.** Capteur PPG selon la revendication 10,

dans lequel la structure d'interface (103) comprenant une paroi médiane (130), entre l'émetteur optique (101) et le détecteur optique (102), et des parois latérales (131) entourant l'émetteur optique (101) et le détecteur optique (102).

12. Capteur PPG selon la revendication 10 ou 11,
dans lequel la structure d'interface (103) est configurée de telle sorte que l'espace (150) soit hermétiquement scellé de l'environnement extérieur.

13. Capteur PPG selon l'une quelconque des revendications 10 à 12, dans lequel la structure d'interface (103) est flexible de telle sorte que la distance entre la base (105) et l'élément d'interface transparent (104) puisse varier lorsqu'une pression est appliquée soit sur la base (105) ou l'élément d'interface transparent (104) ou sur les deux.

14. Capteur PPG selon l'une quelconque des revendications 10 à 13,
dans lequel la structure d'interface (103) est en outre configurée pour empêcher la lumière émise (201) d'atteindre le détecteur optique (102) sans traverser l'élément d'interface transparent (104) et le corps de l'utilisateur (300).

15. Capteur PPG selon la revendication 14,
dans lequel la structure d'interface (103) est en outre configurée pour empêcher la lumière ambiante (206) d'atteindre le détecteur optique (102).

16. Capteur PPG selon l'une quelconque des revendications 1 à 15, configuré pour être porté sur un emplacement du corps (300) d'un utilisateur comprenant l'un quelconque parmi: un brassard, un poignet, le bout d'un doigt ou une oreille ; ou

configuré pour être intégré dans un dispositif portable comprenant l'un quelconque parmi : un bandeau, un bracelet de cheville, un tour de cou, une bague, un casque, un bouchon d'oreille, une aide auditive, un casque, des lunettes, une chemise, un soutien-gorge, un vêtement, un gant, des sous-vêtements, une chaussette, une chaussure, un capteur portable, un patch adhérant à la peau de l'utilisateur, un capteur de signal de pulsatilité ; ou configuré pour être intégré dans un dispositif destiné à être en contact avec le corps de l'utilisateur (300), comprenant l'un quelconque parmi : un capteur de lit, un capteur de chaise, un capteur de toilettes, un capteur de table, un capteur de voiture, un capteur de souris d'ordinateur.

17. Capteur PPG selon l'une quelconque des revendications 1 à 16, configuré pour être porté au poignet et pour déterminer une pression artérielle à partir du signal PPG.

Fig. 1

Fig. 2

Fig. 3

Fig. 4

Fig. 5

Fig. 6

Fig. 7

**EP 3 946 028 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20170360314 A1
- EP 3057138 A
- US 2017311856 A